Europäisches Patentamt

(19) European Patent Office

Office européen des brevets

(11) Publication number: **0 003 898**
**B1**

(12) # EUROPEAN PATENT SPECIFICATION

(45) Date of publication of patent specification: 03.02.82 ·

(51) Int. Cl.³: **C 07 C 131/00** ·

(21) Application number: **79300247.8**

(22) Date of filing: **19.02.79**

(54) Production of acetaldehyde oxime.

(30) Priority: **23.02.78 US 880672**

(43) Date of publication of application:
**05.09.79 Bulletin 79/18**

(45) Publication of the grant of the European patent:
**03.02.82 Bulletin 82/5**

(84) Designated Contracting States:
**DE FR GB IT NL**

(56) References cited:
**NE - A - 70 17568**

(73) Proprietor: **ALLIED CORPORATION**
**Columbia Road and Park Avenue P.O. Box 2245R**
**(Law Dept.)**
**Morristown New Jersey 07960 (US)**

(72) Inventor: **Bonfield, John Henry**
**183 Lyons Road**
**Basking Ridge New Jersey 07920 (US)**
Inventor: **Belsky, Stephen Edward**
**16 Summit Road**
**Parsippany New Jersey 07054 (US)**
Inventor: **Pickens, Donald**
**12 Highfield Circle**
**Mendham New Jersey 07945 (US)**

(74) Representative: **Collier, Jeremy Austin Grey et al,**
**J.A.Kemp & Co. 14, South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

produced tends to increase the acetaldehyde oxime concentration of the vapor and distillation (as shown in the present invention) and the recoverable ammonium sulfate for fertilizer use (as is conventional).

The present invention is not, however, limited to ammonium sulfate-producing oximating reagents but may be practiced with any aqueous hydroxylamine-containing reagent of the type known to the art.

In general, the aqueous oximation reaction mixture contains after reaction between about 1 and about 10 weight percent acetaldehyde oxime, between about 40 and about 90 weight % water and the remainder being salts such as ammonia sulfate and small amounts of ammonium nitrate, free bases such as ammonia and minor organic impurities such as unreacted acetaldehyde and residue.

The aqueous oximation reaction mixture from which distillation occurs may be in one or two phases. With water, acetaldehyde oxime and ammonium sulfate, the existence of a two phase system occurs in a relatively narrow range within the broader range of about 30—60% water, about 0—45% ammonium sulfate and about 2—70% acetaldehyde oxime. The presence of acetaldehyde as an impurity broadens the two phase range somewhat. Distillation from the two phase system is one preferred mode, even though the two phases may disappear once an appreciable amount of acetaldehyde oxime has been distilled off. In the present invention the mixture of acetaldehyde oxime, water and lights is distilled directly from the aqueous oximation reaction mixture rather than trying to distill acetaldehyde oxime from a more refined solution from which the water-soluble materials (such as ammonium sulfate) have been removed. Lights distillation to remove lower boiling impurities such as acetaldehyde and ammonia is conducted on the mixture of acetaldehyde oxime, water and lights after it has been distilled from the aqueous oximation reaction mixture.

Lights distillation from the aqueous oximation reaction mixture before distillation of the acetaldehyde oxime-water mixture is disadvantageous because the concentration of acetaldehyde in the distillate remains at a high level (about 1—3%) falling only slightly. This phenomena is believed to result from the slight decomposition of ammonium sulfate to free ammonia and ammonium acid sulfate and the tendency of ammonium acid sulfate to decompose acetaldehyde oxime to free ammonia and acetaldehyde and other side products. Distilling the overheads from the first distillation (for example 50% acetaldehyde oxime with 2.3% acetaldehyde and 0.6% ammonia in 1750 ml of overheads) produces 4.5%, 2.2% and 1.5% acetaldehyde in the first three 50 ml aliquots and 7.4%, 4.1% and 2.4% ammonia in the first three aliquots. The remaining 1600 mL of overheads from the main distillation contains about 0.78% acetaldehyde (down from 2.3%) and 0.1% ammonia (down from 0.6%), both by weight of acetaldehyde oxime. The overheads from lights distillation may be recycled to the oximation reaction mixture. This lights distillation may occur at 80—96°C for example, with the above data taken from a 20 plate 1:1 reflux ratio batch distillation. Byproducts acetonitrile and alcohol will also preferentially accumulate in the lights overhead. The principal distillation is preferably conducted at between about 96°C and about 100°C.

The oximation reaction itself is performed in a conventional fashion at atmospheric pressure with the temperature at the point where acetaldehyde is added preferably kept below about 50°C and more preferably between about 20°C and 35°C. Once the acetaldehyde is added to the aqueous hydroxylamine containing mixtures of hydroxylamine sulfate and ammonium sulfate, the base (ammonia) is then added, preferably below about 70°C and more preferably between about 20 and about 35°C. In a continuous process, acetaldehyde and base may be added simultaneously.

The product mixture of water and acetaldehyde oxime after lights distillation may be of whatever purity is desired, based on the extent of lights distillation. Purity of less than 1.2% acetaldehyde and less than 0.25% ammonia, based on weight of acetaldehyde oxime, which impurity levels are most suitable for subsequent chlorination, are easily obtained with a relatively small degree of lights distillation.

Example 1 — Demonstration of Azeotrope

Distillations were conducted using the standard Othmer Still technique of the system of acetaldehyde oxime and water with the following values determined: boiling point as a function of the weight fraction acetaldehyde oxime in the binary solution, mol fraction of acetaldehyde oxime in vapor phase as fraction of mol fraction of acetaldehyde oxime in the liquid phase and weight percent and boiling temperature of the azeotrope at pressures from 50—1500 mm Hg absolute. Each of these tests confirmed the existence of an azeotrope at 52.5 weight % acetaldehyde oxime at 95.5°C at 760 mm Hg absolute, whose composition is reduced about 1% acetaldehyde oxime for each 100 mm Hg reduction in pressure.

Example 2 — Ternary System of Ammonium Sulfate,
Acetaldehyde Oxime and Water

The vapor-liquid equilibrium was determined for mixtures of ammonium sulfate, acetaldehyde oxime and water at equilibrium at 760 mm Hg absolute. No appreciable ammonia nor any ammonium salt was present in the vapor phase. Standard Othmer Still methods were used to distill acetaldehyde oxime (AAO) and water from a ternary mixture with ammonium sulfate (AS) and the acetaldehyde oxime concentrations determined by gas chromatography. Data are displayed in Table 1.

TABLE 1

Vapor-Liquid Equilibrium — Acetaldehyde Oxime — Water over
Acetaldehyde Oxime — Water — Ammonium Sulfate

| Liquid Weight % | | | Vapor | |
|---|---|---|---|---|
| Ammonium Sulfate | Wt. % AAO AS Free | Acetaldehyde Oxime | Temp. °C | Wt. % AAO |
| 33.7 | 7.7 | 5.15 | 96.7 | 46 |
| 34.6 | 6.06 | 4.01 | 97.0 | 41.5 |
| 35.3 | 4.38 | 2.78 | 97.5 | 37 |
| 37.0 | 2.99 | 1.88 | 98.0 | 29 |
| 38.5 | 1.99 | 1.23 | 99.0 | 19.5 |
| 40.3 | 1.19 | .71 | 99.0 | 14.5 |
| 42.0 | .74 | .43 | 100.0 | 8.0 |

The balance up to 100% in the liquid and vapor phase was water (except for about 1—3% impurities in the vapor phase including ammonia).

Example 3 — Distillation Process

A reboiler was charged with 185 grams ammonium sulfate, 315 grams water, 100 grams of about 55 weight % acetaldehyde oxime (55.5 grams) in water also containing 0.64% acetaldehyde and 0.22% ammonium by weight of acetaldehyde oxime. At a 10:1 rectification to forward ratio, 15 ml aliquots of forward material was analyzed as shown in Table 2.

TABLE 2

| | | Analysis 15 ml Aliquots O/H | | |
|---|---|---|---|---|
| Time Min. | Overhead °C | Acetaldehyde % vol. Acetal- dehyde Oxime | Weight % Acetaldehyde Oxime | pH Reboiler |
| 0 | 93° | at initial boil up | | 7.6 |
| 25 | 94.5 | 5.25 | 45.7 | 5.6 |
| 45 | 94.5 | 3.4 | 54.3 | 5.5 |
| 70 | 95 | 2.85 | 53.2 | 5.45 |
| 95 | 95 | 2.45 | 54.2 | 4.4 |
| 120 | 95 | 2.0 | 55.5 | 4.1 |
| 140 | 95.5 | 1.7 | 55.5 | 3.95 |
| 165 | 96 | 1.55 | 51 | 3.85 |
| 190 | 96—100 | 1.15 | 35.1 | 3.8 |

It should be appreciated that acetaldehyde oxime was present at only 9.25% of the total original charge or at about 55/415 or about 13.2% based on weight of acetaldehyde oxime and water. Acetaldehyde oxime is present at 45—55.5% in the mixture obtained by distillation until the final period when the temperature was permitted to rise to 100°C.

### Example 4 — Distillation on Total Reflux

Example 3 was repeated with total reflux at 93—94°C and only small samples removed from the reflux for analysis. The proportion of lower boiling acetaldehyde by weight acetaldehyde oxime remained below about 5.5% in the reflux (as measured by gas chromatography) indicating no appreciable decomposition of acetaldehyde oxime to acetaldehyde.

### Example 5 — Continuous Feed

Example 3 was repeated with continuous feed and a three hour average residence time, with the overhead composition at 106°C distillation and a 95°C reboiler heads temperature. The overheads stabilized at 34—35% acetaldehyde oxime and the following impurity proportions by weight of acetaldehyde oxime: 1.8% acetaldehyde and 0.66% ammonia.

It appears from example 5 that the decomposition of ammonium sulfate under distillation conditions to ammonia gas and sulfuric acid lowers the pH of the solution no further than to 3.8 (neutral pH for this mixture would be 5.5) and that such a drop results in no more than 3 weight % acetaldehyde (from product decomposition) relative to acetaldehyde oxime.

### Example 6 — Batch Reaction and Distillation

A mixture of 12800 grams of Rashig hydrox solution containing:

| | |
|---|---|
| $NH_4NO_3$ | 171 grams |
| $H_2SO_4$ | 1097 grams |
| $(NH_4)_2SO_4$ | 2552 grams |
| $(NH_2OH)_2H_2SO_4$ | 7590 grams |

was mixed with 700 grams acetaldehyde (which would stoichiometrically produce 940 grams acetaldehyde oxime) and an aqueous solution of 6 weight % acetaldehyde oxime (80 grams acetaldehyde oxime) to simulate a recycle of the terminal distillation fraction. The mixture was neutralized with 670 grams of ammonia to a pH of 5.5 and then distilled with a 25 plate Oldershaw column at 2:1 reflux to forward ratio. Twenty 250 mL fractions were taken off and analyzed for acetaldehyde oxime by a refractive index method. The first seven fractions contained 50% acetaldehyde oxime and could constitute the "main" or product fraction of the distillate (containing 960 grams acetaldehyde oxime). The next seven 25 mL fractions contained a declining acetaldehyde oxime proportion averaging 5—6% acetaldehyde oxime and could constitute the terminal or recycle fraction (containing 7 grams acetaldehyde oxime). Subsequent 250 mL fractions contained no measurable acetaldehyde oxime and thus need not be subjected to any purification for oxime before ammonium sulfate recovery.

When this example was repeated with multiple recycles of the terminal fraction, the acetaldehyde oxime continued to come off at 90—93% of theoretical yield in a mixture of about 50% acetaldehyde oxime and about 50% water. Even the addition of extra water during recycle had no effect on the product recovery.

When the distillation of this example was repeated by distilling acetaldehyde oxime from a binary system of water and acetaldehyde oxime, the acetaldehyde oxime fell from about 45 weight % in the first 250 mL fraction continuously to about 5 weight % in the twentieth fraction.

### Example 7 — Continuous Reaction and Distillation

The feeds shown in Table 3 (in grams per hour) were made to a microreactor of 250 mL to overflow. Reaction product was fed to a stripping column of 25 plates with 8 inch protruded metal packing with variable reflux return control.

### TABLE 3
### 41°C — Batch Mix Reactor

| Component | Amount Fed |
|---|---|
| Acetaldehyde | 380 grams (mL) of 25% solution in water at a rate of 180 mL/hour (or 95 grams acetaldehyde total) |
| Hydrox Solution | 1601 grams (1275 mL) having 10.7% hydroxylamine by weight as $(NH_2OH)_2H_2SO_4$ |
| Ammonia | 84 grams |

The main cut of distillate (293.5 grams) was determined to contain 43 weight % acetaldehyde oxime by refractive index and 43.5 weight % acetaldehyde oxime by gas liquid chromatography. It also contained as impurities 2.35% acetaldehyde and 1.05% ammonia. In the still bottoms were predominantly ammonium sulfate with a pH of 3.95, about 0.45% excess hydroxylamine and no detectable acetaldehyde oxime. Using an intermediate value of 43.25% acetaldehyde oxime for the "main cut", it could be computed to contain 126.94 grams of acetaldehyde oxime, or 99.8% of theoretical yield.

When this example was repeated in a five hour run, using 880 grams of 25% acetaldehyde oxime and 3770 grams of Raschig hydrox, essentially theoretical yield was obtained in a 608 gram "main cut" at 48.8—50 weight % with only 1.1% acetaldehyde and 2.0% ammonia. The sulfate bottoms had less than 0.02% acetaldehyde oxime and about 0.74% hydroxylamine.

**Claims**

1. Process for preparing acetaldehyde oxime by oximating acetaldehyde with an aqueous oximation reaction mixture which includes a salt and recovering acetaldehyde oxime from the aqueous oximation reaction mixture, characterized in that the acetaldehyde oxime is recovered by first distilling a mixture of acetaldehyde oxime, water and lights including acetaldehyde and ammonia directly from the aqueous oximation reaction mixture and then distilling lights from the said mixture of acetaldehyde oxime, water and lights to remove the lights and produce a product mixture comprising acetaldehyde oxime and water.

2. Process according to claim 1 characterized in that the first distillation is conducted between the boiling point of the azeotrope between acetaldehyde oxime and water and about 100°C.

3. Process according to claim 2 characterized in that the first distillation is conducted between about 96°C and about 100°C.

4. Process according to claim 2 or 3 characterized in that the first distillation is conducted at atmospheric pressure.

5. Process according to any of the preceding claims characterized in that the aqueous oximation reaction mixture includes an ammonium salt.

6. Process according to claim 5 characterized in that the aqueous reaction mixture includes ammonium sulphate.

7. Process according to any of the preceding claims characterized in that the aqueous oximation reaction mixture used is made by addition of a base to an aqueous hydroxylamine-containing solution including hydroxylamine sulphate, sulphuric acid and ammonium sulphate.

8. Process according to claim 7 characterized in that the base used is ammonia.

9. Process according to any of the preceding claims characterized in that the product mixture contains less than 1.2% acetaldehyde and less than 0.25% ammonia, based on weight of acetaldehyde oxime.

10. Process according to any of the preceding claims characterized in that the mixture of acetaldehyde oxime and water is reacted after the lights distillation with $Cl_2$.

11. Process according to claim 10 wherein the mixture of acetaldehyde oxime and water is diluted after the lights distillation and then reacted with $Cl_2$.

**Patentansprüche**

1. Verfahren zur Herstellung von Acetaldehydoxim durch Oximierung von Acetaldehyd mit einem wäßrigen Oximierungs-Reaktionsgemisch, das ein Salz enthält, und Gewinnung von Acetaldehydoxim aus dem wäßrigen Oximierungs-Reaktionsgemisch, dadurch gekennzeichnet, daß das Acetaldehydoxim gewonnen wird, indem man zunächst ein Gemisch von Acetaldehydoxim, Wasser und leicht flüchtigen Komponenten einschließlich Acetaldehyd und Ammoniak direkt aus dem wäßrigen Oximierungs-Reaktionsgemisch abdestilliert und dann von diesem Gemisch von Acetaldehydoxim, Wasser und leicht flüchtigen Komponenten die leicht flüchtigen Komponenten abdestilliert und so diese leicht flüchtigen Komponenten entfernt und ein Acetaldehydoxim und Wasser umfassendes Produktgemisch erzeugt.

2. Verfahren nach Anspruch 1, dadurch gekennzeichnet, daß man die erste Destillation zwischen dem Siedepunkt des Azeotrops von Acetaldehydoxim mit Wasser und etwa 100°C durchführt.

3. Verfahren nach Anspruch 2, dadurch gekennzeichnet, daß man die erste Destillation zwischen etwa 96°C und etwa 100°C durchführt.

4. Verfahren nach Anspruch 2 oder 3, dadurch gekennzeichnet, daß man die erste Destillation bei Atmosphärendruck durchführt.

5. Verfahren nach einem der vorausgehenden Ansprüche, dadurch gekennzeichnet, daß das wäßrige Oximierungs-Reaktionsgemisch ein Ammoniumsalz enthält.

6. Verfahren nach Anspruch 5, dadurch gekennzeichnet, daß das wäßrige Oximierungs-Reaktionsgemisch Ammoniumsulfat enthält.

7. Verfahren nach einem der vorausgehenden Ansprüche, dadurch gekennzeichnet, daß das verwendete wäßrige Oximierungs-Reaktionsgemisch durch Zugabe einer Base zu einer wäßrigen

hydroxylaminhaltigen Lösung, die Hydroxylaminsulfat, Schwefelsäure und Ammoniumsulfat enthält, erhalten wird.

8. Verfahren nach Anspruch 7, dadurch gekennzeichnet, daß die verwendete Base Ammoniak ist.

9. Verfahren nach einem der vorausgehenden Ansprüche, dadurch gekennzeichnet, daß das Produktgemisch weniger als 1,2% Acetaldehyd und weniger als 0,25% Ammoniak, bezogen auf das Acetaldehydoximgewicht, enthält.

10. Verfahren nach einem der vorausgehenden Ansprüche, dadurch gekennzeichnet, daß das Gemisch von Acetaldehydoxim und Wasser nach der Abdestillation der leicht flüchtigen Komponenten mit Cl$_2$ umgesetzt wird.

11. Verfahren nach Anspruch 10, dadurch gekennzeichnet, daß das Gemisch von Acetaldehydoxim und Wasser nach der Abdestillation der leicht flüchtigen Komponenten verdünnt und dann mit Cl$_2$ umgesetzt wird.

**Revendications**

1. Procédé de préparation d'oxime d'acétaldéhyde en oximant de l'acétaldéhyde avec un mélange aqueux de réaction d'oximation qui comprend un sel et en récupérant l'oxime d'acétaldéhyde à partir du mélange aqueux de réaction d'oximation, caractérisé en ce que l'oxime d'acétaldéhyde est récupéré en distillant d'abord un mélange d'oxime d'acétaldéhyde, d'eau et de produits légers comprenant de l'acétaldéhyde et de l'ammoniac directement à partir du mélange aqueux de réaction d'oximation, et puis en distillant les produits légers à partir de ce mélange d'oxime d'acétaldéhyde, d'eau et de produits légers pour retirer les produits légers et fournir un mélange de produit comprenant de l'oxime d'acétaldéhyde et de l'eau.

2. Procédé selon la revendication 1, caractérisé en ce que la première distillation est conduite entre le point d'ébullition de l'azéotrope entre l'oxime d'acétaldéhyde et l'eau et environ 100°C.

3. Procédé selon la revendication 2, caractérisé en ce que la première distillation est conduite entre environ 96°C et environ 100°C.

4. Procédé selon la revendication 2 ou la revendication 3, caractérisé en ce que la première distillation est conduite sous la pression atmosphérique.

5. Procédé selon l'une quelconque des revendications précédentes, caractérisé en ce que le mélange aqueux de réaction d'oximation comprend un sel d'ammonium.

6. Procédé selon la revendication 5, caractérisé en ce que le mélange aqueux de réaction d'oximation comprend du sulfate d'ammonium.

7. Procédé selon l'une quelconque des revendications précédentes, caractérisé en ce que le mélange aqueux de réaction d'oximation utilisé est réalisé par addition d'une base à une solution aqueuse contenant de l'hydroxylamine, renfermant du sulfate d'hydroxylamine, de l'acide sulfurique et du sulfate d'ammonium.

8. Procédé selon la revendication 7, caractérisé en ce que la base utilisée est de l'ammoniac.

9. Procédé selon l'une quelconque des revendications précédentes, caractérisé en ce que le mélange de produits contient moins de 1,2% d'acétaldéhyde et moins de 0,25% d'ammoniac, en se basant sur le poids de l'oxime d'acétaldéhyde.

10. Procédé selon l'une quelconque des revendications précédentes, caractérisé en ce que le mélange d'oxime d'acétaldéhyde et d'eau est mis à réagir après la distillation des produits légers avec Cl$_2$.

11. Procédé selon la revendication 10, caractérisé en ce que le mélange d'oxime d'acétaldéhyde et d'eau est dilué après la distillation des produits légers et puis mis à réagir avec Cl$_2$.